# FASCICULE DE BREVET EUROPEEN

(11) **EP 1 336 402 B1**
(45) Date de publication et mention de la délivrance du brevet: **29.06.2005**
(21) Numéro de dépôt: 03290392.4
(22) Date de dépôt: 18.02.2003
(51) Int. Cl.: A61K 7/06

(54) **Association de diguanosine tetraphosphate et de dérivés nicotiniques, destinées aux traitements des désordres capillaires, notamment pour lutter contre la chute des cheveux**
Diguanosintetraphosphate und Nikotinderivate enthaltende Zusammensetzung zur Behandlung von Haarstörungen, insbesondere zur Bekämpfung von Haarausfall
Association of diguanosine tetraphosphate and nicotinic derivatives used for the treatment of capillary disorders, in particular the treatment of hair loss

(30) Priorité: 19.02.2002 FR 0202073
(43) Date de publication de la demande: 20.08.2003
(73) Titulaire: Pierre Fabre Dermo-Cosmetique, 92100 Boulogne-Billancourt (FR)
(72) Inventeur: Fort-Lacoste, Lydie, 31860 Labarthe sur Leze (FR); Jeanjean, Michel, 31320 Castanet Tolosan (FR)
(74) Mandataire: Warcoin, Jacques

(56) Documents cités:
- WO-A-99/38483
- PATENT ABSTRACTS OF JAPAN vol. 1999, no. 13, 30 novembre 1999 (1999-11-30) & JP 11 236319 A (SHISEIDO CO LTD), 31 août 1999 (1999-08-31)

## Description

La présente invention se rapporte à de nouvelles compositions dermo-cosmétologiques pour la stimulation de la croissance capillaire et le traitement de l'alopécie, ainsi qu'à des méthodes de traitement de ces désordres capillaires.

### RAPPEL SUR LA CHUTE DES CHEVEUX

Le terme d'alopécie résume l'ensemble des troubles atteignant le follicule pileux, son environnement ainsi que le cycle de croissance de la tige pilaire et ayant pour conséquence la perte occasionnelle ou définitive de tout ou partie des cheveux.

On citera les 2 types d'alopécies les plus répandues, l'Alopécie Aérata et l'Alopécie Androgénogénétique ou Androgénique. On peut aussi citer l'alopécie induite par certains médicaments utilisés en thérapie anti-cancéreuse.

On peut également citer l'Alopécie aiguë généralisée, qui se rapporte à une chute rapide d'une grande quantité de cheveux, à la suite d'un stress intense (divorce, choc psychologique, décès), une grossesse, une opération chirurgicale, une maladie générale, une chimiothérapie. Tous les cheveux se mettent en repos, tombent au bout de trois mois puis repoussent si la cause du stress disparaît. Outre les médicaments anticancéreux qui déclenchent une chute importante des cheveux, ceux utilisés contre le cholestérol, l'acné et l'hypertension peuvent provoquer une alopécie bénigne le temps que dure le traitement.

Il existe également l'Alopécie localisée, qui peut être due à une lésion définitive du cuir chevelu (radiothérapie ou brûlure), à une maladie de peau (lichen plan, lupus érythémateux, tumeurs de la peau) ou à la pelade (zone habituellement pileuse dépourvue de tout poil, mais d'apparence normale) qu'on considère liée à des facteurs psychologiques. La pelade universelle (perte de tous les poils du corps) est très rare. En cas de pelade, la repousse est possible, mais incertaine.

Depuis longtemps, les scientifiques recherchent à travers différentes voies biologiques, les solutions visant à stimuler la croissance du cheveu ou à prolonger son ancrage au cuir chevelu. Cette préoccupation est d'autant plus marquée dans le domaine de l'alopécie androgénique et plus particulièrement fréquente chez les sujets masculins, chez lesquels on constate une perturbation du cycle de croissance pilaire, entraînant la chute du cheveu.

Le follicule pileux constitue l'unité biologique fondamentale, directement impliquée dans le développement et le maintien du système pilaire. Il rassemble différentes entités structurales telles que la papille dermique, la glande sébacée, les gaines épithéliales, l'innervation ou la vascularisation impliquées séparément ou conjointement dans le cycle de développement des cheveux. Le lien existant entre la papille dermique et le bulbe reste déterminant dans la croissance du cheveu (cf. Oliver RF. Histological studies of whisker regeneration in the hooded rat. J Embryol Exp Morphol 1966 ; 311. 560-2 et Jahoda CAB. Horne KA. Oliver RF. Induction of hair growth by implantation of cultured dermal papilla cells. Nature 1966; 311. 560-2).

Le cycle de croissance pilaire est représenté dans le temps par trois phases dont la plus importante est la phase anagène ou phase de croissance. Son accélération induite par différentes causes biologiques entraîne le raccourcissement de la vie du cheveu et précipite son entrée en phase catagène.

D'une façon générale, l'alopécie relève de causes multifactorielles.

En particulier, il est reconnu aujourd'hui l'intervention de différents facteurs génétiques dans sa mise en place chez l'adulte jeune.

La composante hormonale joue un rôle prépondérant et sûrement décisif dans l'installation et l'entretien de ces troubles. En effet une dérégulation hormonale d'origine endocrine entraîne une production excessive de testostérone dont l'hydroxylation sera catalysée par la 5-a-réductase, en dihydrotestostérone active. Ce métabolite est doté d'une activité androgénique importante qui lui permet d'agir à deux niveaux :
- sur la glande sébacée dont le développement exagéré induit une production excessive de sébum conduisant à une hyperséborrhée,
- sur les cellules de la papille dermique entraînant un raccourcissement de la phase anagène et nécessairement un épuisement du follicule pileux.

Le facteur vasculaire constitue également un paramètre fondamental pour la croissance et le développement du follicule pileux.

En effet, la papille dermique, structure contrôlant la croissance pilaire, se caractérise par la présence d'un réseau vasculaire (cf. Richard A. Ellis, Guiseppe Moretti. Vascular patterns associated with catagen hair follicules in the human scalp. Annals New York Academy of Sciences, 1959-83, 448-547) très développé en phase anagène. Il a été démontré que les cellules de cette papille expriment durant ce stade et de façon intense, un facteur de croissance angiogénique le VEGF (S.Lachgar M.Charveron in "Hair for the nex millenium" - EdS. D, JJ Van Neste - VA Randall - H. Baden - H. Ogawa et R. Oliver - Elsevier - Amsterdam - 1996 - p. 407-412).

Ce facteur de croissance vasculaire endothélial permet le maintien de la vascularisation nécessaire à la croissance du néo-cheveu et son développement pendant toute la phase anagène.

Donc, les molécules susceptibles d'induire une angiogénèse sont des candidats de choix dans l'induction et le maintien de la vascularisation au niveau de la papille dermique.

Cette découverte est d'autant plus importante qu'il y a disparition du réseau capillaire durant le passage du stade anagène au stade catagène. Ainsi, le développement pilaire semble être lié au maintien des capillaires sanguins puisque la chute du poil est associée une disparition du réseau capillaire de la papille durant la phase télogène.

Ceci a fait l'objet de brevets d'applications Pierre Fabre Dermo-Cosmétique, notamment par l'utilisation d'extrait de Ruscus aculeatus (Brevet FR 2779348) et d'extrait de Pfaffia paniculata (Brevet FR 2779052) pour stimuler la production de VEGF.

### LES DERIVES NICOTINIQUES

L'Acide Nicotinique (Acide 3-Pyridine carboxylique) 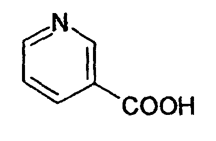 désigné comme facteur vitaminique PP, est décrit comme co-facteur enzymatique vitaminique. Il a été reconnu comme possédant une activité préventive et curative dans le traitement de la pellagre chez les chiens.

Parmi ses dérivés, on peut aussi citer l'amide nicotinique ou vitamine PP (Nicotinamide) jouant un rôle important dans les réactions métaboliques, énergétiques, notamment les réactions d'oxydo-réductions. Elle participe de façon active à la chaîne respiratoire et à la fourniture d'énergie dans de nombreuses réactions biochimiques.

Son implication directe dans la bonne santé de la peau et des phanères en fait une vitamine de choix dans le traitement des désordres capillaires au sens large.

Parmi les dérivés de l'acide nicotinique, on peut citer les esters dont les plus connus sont les nicotinates de méthyle, d'éthyle et de benzyle. Ces esters sont largement décrits et documentés comme accélérateurs de la circulation sanguine et notamment la circulation périphérique en raison de leur effet rubéfiant.

Parmi les esters de l'acide nicotinique, l'un des plus intéressants semble être l'ester de l'alpha tocophérol ou d-l-alpha tocophéryl nicotinate. Son intérêt est double dans la mesure où il peut combiner les effets de l'alpha tocophérol (Vitamine E) et de l'Acide nicotinique.

L'Alpha Tocophéryl Nicotinate (nicotinate de tocophérol, αTN), de poids moléculaire voisin de 535, répond à la formule développée ci-après (C35H53NO3).

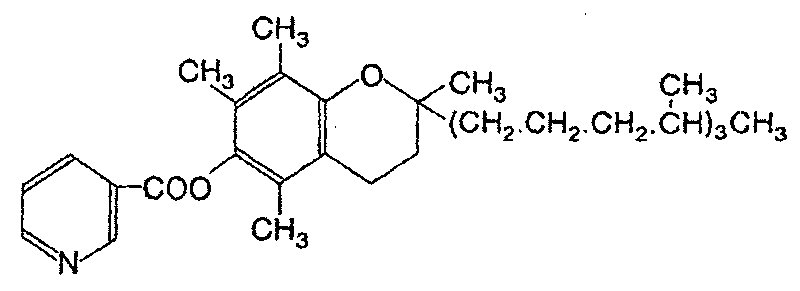

L'αTN favorise l'irrigation sanguine de l'épiderme dans les applications topiques en accélérant la circulation du sang grâce à son effet vasodilateur.

Outre son activité sur la circulation sanguine, l'αTN présente une activité stimulatrice des cellules du follicule pileux (Journal of Chromatography 593, 1992, 95-97). Cette double activité en fait par conséquence un principe actif à usage topique intéressant pour traiter les désordres capillaires tels que l'alopécie, la canitie ou le vieillissement.

A titre d'exemples, on peut citer deux articles publiés dans la revue américaine COSMETICS & TOILETRIES - Vol. 102 - 51-59 (1987) - Vol. 108 - 79-94 (1993). Ces deux publications mettent en exergue l'intérêt de son activité stimulante sur la petite circulation pour des applications topiques cutanées et capillaires en cosmétologie.

Une étude effectuée par analyse thermique cutanée (Thermal Clearance Method) montre que l'effet accélérateur sur la circulation sanguine du petit vaisseau est obtenu dans le cas de l'αTN sans présenter les effets secondaires indésirables observés avec les autres esters nicotiniques.

En effet, les esters plus connus tels que nicotate d'ethyle ou de methyle présentent une activité rubéfiante marquée se traduisant par un échauffement local et une sensation d'irritation pouvant être traduite comme une perception de brûlure. (FGM VOGEL « Tocopheryl Nicotinate, An active ingredient in cosmetics - IFSCC - 1986 - Barcelone). Ainsi, l'α-TN est un additif souvent utilisé en cosmétique.

Parmi l'analyse documentaire des brevets, on peut citer à titre d'exemples deux brevets de SHISEIDO JP 11236319 A et JP 11302131 A présentant les associations de l'acide nicotinique ou de l'ester avec l'acide hyaluronique et le Minoxidil comme actives sur la circulation sanguine.

### LE DIGUANOSINE TETRAPHOSPHATE OU GP4G

Il s'agit d'un principe actif de biotechnologie marine extrait de zooplancton : *Artemia Salina,* et répondant à la structure chimique suivante :

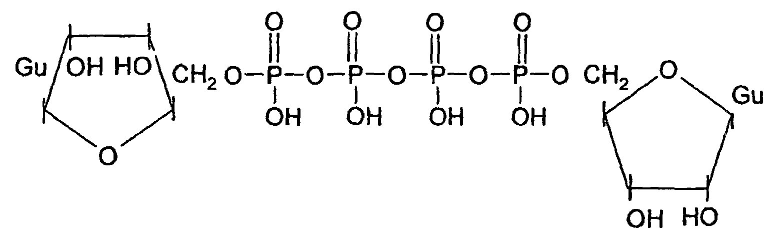

*Artemia Salina* est un petit crustacé vivant habituellement dans les étangs saumâtres. Son cycle biologique présente la particularité de pouvoir passer par une phase de dormance lorsque les conditions environnementales sont critiques (ARTEMIA SALINA ET L'EXPRESSION DES MESSAGERS - LE GAL Y - BIOCHIMIE MARINE - Ed. MASSON - 1988 - pp. 177-181).

Ce que l'on appelle improprement des « oeufs » d'Artemia sont en réalité des gastrula dormantes ou enkystées susceptibles de rester dans cet état pendant plusieurs années. Ces gastrula enkystées contiennent une très grande quantité d'un nucléotide atypique, le diguanosine tétraphosphate (ou GP4G).

Lorsque l'environnement redevient plus clément, Artemia reprend son développement, en utilisant le GP4G pour redémarrer ses activités métaboliques. Le GP4G diminue rapidement et l'ATP augmente, ce qui suggère une conversion du GP4G en ATP.

GP4G étant une molécule ubiquitaire, de nombreuses cellules de mammifères possèdent le matériel enzymatique nécessaire à l'assimilation du GP4G. Ainsi, dans l'art antérieur, des extraits d'Artemia Salina ont été utilisés pour régénérer le tissu cutané (brevet COTY WO 9938483) ou pour protéger le cuir chevelu des attaques radicalaires (Brevet COTY EP 1025835).

De façon inattendue, la Demanderesse a mis en évidence un effet angiogène pour le GP4G, ainsi qu'un effet synergique sur les propriétés angiogéniques lorsque l'on associe le nicotinate de tocophérol avec du GP4G. Le Demanderesse a également montré que de telles propriétés peuvent être utilisées pour favoriser la croissance pilaire.

Ainsi, la présente invention se rapporte à une composition comprenant du nicotinate de tocophérol, en association avec du GP4G.

La composition selon l'invention contient les deux principes actifs précités en quantité suffisante pour obtenir l'effet recherché contre l'alopécie, et qui peuvent facilement être déterminées par l'homme du métier en fonction des valeurs données dans les exemples de la présente demande. Ainsi, la composition selon l'invention contient les deux principes actifs en quantité suffisante pour stimuler l'angiogenèse, et/ou pour stimuler la croissance des cheveux et/ou pour diminuer la chute des cheveux.

De préférence, cette composition contient en outre des excipients cosmétologiquement acceptables, et il s'agit d'une composition cosmétique. En effet, du fait l'alopécie présente plutôt des désagréments d'ordre esthétiques, on ne peut généralement pas la considérer comme une pathologie, nécessitant un traitement. Toutefois, l'aspect cosmétique peut s'avérer très important notamment dans les relations sociales.

Dans un autre mode de réalisation, la composition selon l'invention contient en outre des excipients pharmacologiquement acceptables, et qu'il s'agit d'une composition pharmaceutique. On utilise alors la composition selon l'invention en combinaison avec d'autres compositions pharmaceutiques, destinées à lutter contre les causes de l'alopécie (médicaments anti-stress, hypocholestérolémique, anti-acné...). Toutefois, et même dans ce cas, le but principal d'utilisation de la composition selon l'invention est d'améliorer l'apparence esthétique et cosmétique du patient.

Dans un mode de réalisation préféré, la composition selon l'invention est formulée pour une application topique externe.

L'homme du métier connaît les excipients permettant de préparer les compositions selon l'invention, pour un usage topique externe. Les exemples illustrent certaines compositions appropriées à un tel usage.

Dans un autre mode de mise en oeuvre, l'invention se rapporte à des produits contenant du nicotinate de tocophérol et du GP4G comme produit de combinaison pour une utilisation simultanée, séparée ou étalée dans le temps pour le traitement de l'alopécie. En effet, dans un mode de réalisation particulier de l'invention (exemple 2.2), les produits selon l'invention sont destinés à une utilisation étalée dans le temps, et se présentent sous la forme d'une première solution (contenant les deux actifs) à appliquer (de préférence le soir), et d'une seconde solution (contenant un seul des actifs) à appliquer entre 8 et 12 heures suivant l'application de la première solution (de préférence le matin).

Les produits selon l'invention sont de préférence formulés pour un usage topique externe.

L'invention se rapporte également à l'utilisation cosmétique de nicotinate de tocophérol en association avec du GP4G pour le traitement de l'alopécie, des désordres capillaires, ou pour favoriser la pousse capillaire.

Dans un autre mode de réalisation, l'invention se rapporte également à l'utilisation de nicotinate de tocophérol en association avec du GP4G pour la préparation d'un médicament destiné au traitement de l'alopécie.

Enfin, l'invention se rapporte à une méthode de traitement cosmétique pour favoriser la pousse capillaire et/ou diminuer l'alopécie, comprenant l'étape d'administrer du nicotinate de tocophérol et du GP4G à un sujet.

Dans un mode de réalisation préféré, on lesdits composés sont administrés par voie topique externe.

Dans un mode de réalisation particulier, lesdits composés sont administrés de manière simultanée. Dans un autre mode de réalisation, lesdits composés sont administrés de manière séparée ou étalée dans le temps.

Dans un mode de réalisation préféré, le nicotinate de tocophérol est présent en une quantité comprise entre 0,05 % (en poids) et 5% de la composition, ou des produits selon l'invention. Dans un mode de réalisation plus préféré, le nicotinate de tocophérol est présent en une quantité comprise entre 0,1% et 2,5% de la composition, ou des produits selon l'invention.

Dans un mode de réalisation préféré, le GP4G est présent en une quantité comprise entre 0,01 % (en poids) et 5% de la composition, ou des produits selon l'invention. Dans un mode de réalisation plus préféré, le nicotinate de tocophérol est présent en une quantité comprise entre 0,05% et 2,5% de la composition, ou des produits selon l'invention.

Les exemples qui suivent sont destinés à illustrer certains modes de réalisation particuliers de l'invention et représentent en particulier certaines compositions utilisables pour la mise en oeuvre de l'invention. Les excipients mentionnés dans les exemples de composition ne sont donnés qu'à titre illustratif; et il est à la portée de l'homme du métier d'en substituer d'autres.

### EXEMPLES

### Exemple 1 : synergie d'activité du GP4G et nicotinate de tocophérol

On a observé une augmentation fonction-dose, du métabolisme des cellules de la papille dermique (CPD) en présence de GP4G, ce qui traduit l'intérêt de ce principe actif dans la stimulation de la croissance pilaire.

Par ailleurs, la composante vasculaire jouant un rôle fondamental dans la croissance du follicule pileux, l'influence des principes actifs précédemment décrits sur ce paramètre a été évaluée sur un modèle d'angiogénèse in vitro.

La source de GP4G utilisée est issue de biotechnologie marine, commercialisée par la Société Vincience Seporga (http://www.mmpinc.com/seporga.htm).

Le modèle repose sur une co-culture de cellules endothéliales humaines et de fibroblastes humains (kit angiogénèse Bioprédic International), qui est réalisée en présence ou pas des actifs à tester.

Les cellules endothéliales migrent à travers la matrice pour former des structures tubulaires, qui sont quantifiées au bout de 12 jours de culture. La quantification de la longueur des capillaires est réalisée par analyse d'image.

| | LONGUEUR DES TUBULES PAR CHAMP DE COMPTAGE | |
|---|---|---|
| | Moyenne | Pourcentage d'augmentation/témoin |
| Témoin non traité | 4150 | - |
| Ref. positive (VEGF 10ng/ml) | 5294 | + 28 % |
| Ref. Négative (Suramine 20 µM) | 3072 | - 26 % |
| Minoxidil 25 µg/ml | 7662 | + 85 % |
| GP4G 0.01 % | 3814 | - |
| 0.1 % | 4040 | - |
| 1.0 % | 6803 | + 64 % |
| Niconitate de tocophérol | | |
| 1 µg/ml | 4015 | - |
| 10 µg/ml | 4634 | **+ 12 %** |
| 20 µg/ml | 5363 | + 29 % |
| GP4G (0.01 %) + Nicotinate de tocophérol (10 µg/ml) | 5107 | **+ 23 %** |

Il ressort de ceci que le GP4G et le nicotinate de tocophérol présentent tous deux un pouvoir angiogénique, et que que l'on peut observer un effet synergique lorsque l'on associe ces deux actifs.

L'intérêt de l'association de ces deux actifs a été confirmé sur d'autres modèles cellulaires, notamment sur le métabolisme des cellules de la papille dermique du follicule pileux en culture mono-couche, où l'association GP4G + nicotinate de tocophérol a montré une augmentation de + 26,2 % du métabolisme cellulaire, supérieure à celle des actifs testés seuls (12,6 % et 18,3 % respectivement).

Enfin, l'intérêt de l'association GP4G + Nicotinate de tocophérol a été confirmée en application topique sur un explant cutané, avec une augmentation de la consommation en glucose de + 39 %.

L'ensemble de ces résultats permettent de conclure à l'intérêt d'associer le GP4G et le Nicotinate de tocophérol pour améliorer le métabolisme cellulaire et la vascularisation, permettant ainsi d'avoir un rôle favorable dans la croissance pilaire, notamment dans la lutte contre l'alopécie et la pelade et la prévention de la canitie.

### Exemple 2 : exemples de formulations topiques à usage capillaire

### 2.1 - Lotion Triphasique anti-alopécie

| Phase poudre (A) | (2.5 %) |
|---|---|
| β et γ cyclodextrines (50/50) | 0.8 % |
| Silice pyrrogénée | 0.06 % |
| Lactoserum poudre | 1.7 % |

| Phase lipophile (B) | (35 %) |
|---|---|
| Extrait huileux de serenoa repens | QS |
| Alpha tocopheryl nicotinate | 1.5 % |
| Linoléate d'éthyle | QS |
| Alpha (-) Pinène | QS |
| Ethanol | QS |
| C12-C15 Alkyl benzoates | QS |

| Phase hydrophile (C) | (62.5 %) |
|---|---|
| Nicotinamide | QS |
| Lactamide MEA | QS |
| Arginine | QS |
| Vitamine B6 (chlorhydrate) | QS |
| Gluconate de zinc | QS |
| Piroctonolamine | QS |
| Alcool isopropylique | QS |
| Eau déminéralisée stérile | QS |
| Diguanosine tétraphosphate solution (GP4G) | 2 % |

### 2.2 - Chronotraitement pour la chute des cheveux

| *Solution à appliquer le soir* | |
|---|---|
| Extrait de Ruscus 957 | 7 % |
| Diguanosine Tetraphosphate solution | 2 % |
| Alpha-tocophéryl nicotinate | 0.5 % |
| Piroctonolamine | 0.2 % |
| Hexylène glycol | QS |
| Parfum | QS |
| Ethanol | QS |
| Eau déminéralisée stérile | QSP 100 g |

| *Solution à appliquer le matin* | |
|---|---|
| Extrait lipostérolique de Sabal | 0.4 % |
| Acide laurique | 0.6 % |
| Acide b-glycyrrhétinique | 0.30 % |
| a-Tocophéryl Nicotinate | 0.15 % |
| Décamethylcyclopentasiloxane | QS |
| Ethyl hexyl palmitate | QS |
| Ethylphtalate | QS |
| Parfum | QS |
| Ethanol | QSP 100 g |

### 2.3 - Sérum antichute - antivieillissement

| | |
|---|---|
| Extrait de Ruscus 957 | 2 % |
| D-Panthenol | 0.4 % |
| Pyridoxine Chlorhydrate | 0.10 % |
| Zinc gluconate | 0.25 % |
| Phytoflavones | 1 % |
| Extrait de quinquina | 0.75 % |
| a-Tocophéryl Nicotinate | 0.15 % |
| Diguanosine Tetraphosphate solution | 1 % |
| PEG 40 hydrogenated Castor Oil | 0.60 % |
| Ethoxydiglycol | 2 % |
| Eau déminéralisée stérile | QSP 100 g |

### 2.4 - Soin crème après shampooing antivieillissement

| | |
|---|---|
| Extrait glycolique de Pfaffia | 3 % |
| Hydrolysat de protéines de soie (sol. 20 %) | 2 % |
| Behenyltrimmonium chlorure | QS |
| Cyclomethicone | QS |
| Beurre de karité | QS |
| Hydroxypropyltrimmonium Guar | QS |
| Parfum | QS |
| a-Tocophéryl Nicotinate | 0.3 % |
| Diguanosine Tetraphosphate solution Acide citrique pH 4.5 | 1.5 % |
| Eau déminéralisée stérile | QSP 100 g |

### 2.5 - Shampooing crème relais-antichute

| | |
|---|---|
| a-Tocophéryl Nicotinate | 0.5 % |
| Diguanosine Tetraphosphate solution | 1.5 % |
| D-Panthenol | 0.3 % |
| Sodium Laureth Sulfate (28 %) | 25 % |
| Sodium cocoamphoacetate (30 %) | 12 % |
| Cocamidopropyl betaïne (30 %) | 7 % |
| PEG 7 Glyceryl cocoate | QS |
| PEG 150 distearate | QS |
| Glycol palmitate | 0.75 % |
| Ethylèneglycol distearate | 0.75 % |
| Hydroxypropyl Guar Acide citrique pH 6.5 | 0.075 % |
| Conservateurs | QS |
| Eau déminéralisée stérile | QSP 100 g |

### 2.6 - Lotion capillaire usage fréquent

| | |
|---|---|
| a-Tocophéryl Nicotinate | 0.2 % |
| Diguanosine Tetraphosphate solution | 0.75 % |
| Acétamide MEA | 0.75 % |
| Sulfate de Zinc | 1 % |
| D-Panthénol | 0.15 % |
| Pyridoxine chlorhydrate | 0.20 % |
| Dimethicone copolyol | QS |
| Alcool 96% vol. | 35 % vol |
| Eau déminéralisée stérile | QSP 100 g |

### 2.7 - Traitement antichute

| | |
|---|---|
| Acide b glycyrrhetinique | 0.3 g |
| Hexylene glycol | 10 g |
| Alcool à 96 % vol. | 35 g |
| Diethylphtalate | 0.50 g |
| Chlorure de sodium | 0.1 g |
| Diméthicone copolyol | 0.1 g |
| Extrait fluide de Pfaffia | 4 g |
| Diguanosine tétraphosphate lyophilisat | 0.05 g |
| Eau déminéralisée stérile | 5 g |
| α-tocophérol nicotinate | 1 g |
| Crotamiton | 0.50 g |
| Extrait de courge hydrolysé | 2 g |
| Cyclometicone | QSP 100 g |

### 2.8 - Gel rubéfiant pour traiter la pelade

| | |
|---|---|
| α tocophéryl nicotinate | 2.2 g |
| Diguanosine tetraphosphate Atomisat | 0.3 g |
| Huile essentielle de Cèdre | 0.2 g |
| Huile essentielle de Niaouli | 0.15 g |
| PEG-40 Hydrogenated Castor Oil | 1.75 g |
| Ethoxydiglycol | 7.0 g |
| Carbomer | QSP |
| Aminomethyl-propanol | QSP |
| Eau déminéralisée | 15 g |
| Ethanol | QSP 100 g |

## Revendications

1. Composition comprenant du nicotinate de tocophérol, en association avec du GP4G.

2. Composition selon la revendication 1, **caractérisée en ce qu'**elle est formulée pour un usage topique externe.

3. Produits contenant du nicotinate de tocophérol et du GP4G comme produit de combinaison pour une utilisation simultanée, séparée ou étalée dans le temps pour le traitement de l'alopécie.

4. Produits selon la revendication 3, **caractérisé en ce qu'**ils sont formulés pour un usage topique externe.

5. Utilisation cosmétique de nicotinate de tocophérol en association avec du GP4G pour le traitement de l'alopécie.

6. Méthode de traitement cosmétique pour favoriser la pousse capillaire et/ou diminuer l'alopécie, comprenant l'étape d'administrer du nicotinate de tocophérol et du GP4G à un sujet.

7. Méthode selon la revendication 6, **caractérisée en ce que** l'on applique lesdits composés par voie topique externe.

8. Méthode selon la revendication 6 ou 7, **caractérisée en ce que** l'on applique lesdits composés de manière simultanée.

9. Méthode selon la revendication 6 ou 7, **caractérisée en ce que** l'on applique lesdits composés de manière séparée ou étalée dans le temps.

## Patentansprüche

1. Zusammensetzung, umfassend Tocopherolnicotinat in Kombination mit GP4G.

2. Zusammensetzung nach Anspruch 1, **dadurch gekennzeichnet, dass** sie für eine äußerliche topische Anwendung formuliert ist.

3. Produkte, welche Tocopherolnicotinat und GP4G enthalten, als Kombinationsprodukt für eine gleichzeitige, getrennte oder zeitlich gestaffelte Verwendung für die Behandlung von Alopezie.

4. Produkte nach Anspruch 3, **dadurch gekennzeichnet, dass** sie für eine äußerliche topische Anwendung formuliert sind.

5. Kosmetische Verwendung von Tocopherolnicotinat in Kombination mit GP4G für die Behandlung von Alopezie.

6. Kosmetisches Behandlungsverfahren, um den Haarwuchs zu begünstigen und/oder Alopezie zu verringern, welches den Schritt umfasst, Tocopherolnicotinat und GP4G an ein Subjekt zu verabreichen.

7. Verfahren nach Anspruch 6, **dadurch gekennzeichnet, dass** man die Verbindungen auf äußerlichem topischem Wege anwendet.

8. Verfahren nach Anspruch 6 oder 7, **dadurch gekennzeichnet, dass** man die Verbindungen gleichzeitig anwendet.

9. Verfahren nach Anspruch 6 oder 7, **dadurch gekennzeichnet, dass** man die Verbindungen getrennt oder zeitlich gestaffelt anwendet.

## Claims

1. Composition comprising tocopherol nicotinate, in combination with GP4G.

2. Composition according to Claim 1, **characterized in that** it is formulated for external topical use.

3. Products comprising tocopherol nicotinate and GP4G as combination product for simultaneous use, separate use or use spread out over time in the treatment of alopecia.

4. Products according to Claim 3, **characterized in that** they are formulated for external topical use.

5. Cosmetic use of tocopherol nicotinate in combination with GP4G in the treatment of alopecia.

6. Cosmetic treatment method for promoting hair growth and/or reducing alopecia, comprising the stage of administering tocopherol nicotinate and GP4G to a subject.

7. Method according to Claim 6, **characterized in that** the said compounds are applied via the external topical route.

8. Method according to Claim 6 or 7, **characterized in that** the said compounds are applied simultaneously.

9. Method according to Claim 6 or 7, **characterized in that** the said compounds are applied separately or in a way spread out over time.
